# EUROPÄISCHE PATENTSCHRIFT

(11) **EP 2 758 118 B1**
(45) Veröffentlichungstag und Bekanntmachung des Hinweises auf die Patenterteilung: **08.07.2015**
(21) Anmeldenummer: 12778038.5
(22) Anmeldetag: 21.09.2012
(51) Int. Cl.: A61N 1/05, A61N 1/08, A61B 17/00

(54) **MEDIZINISCHE VORRICHTUNG ZUM EINBRINGEN IN DEN MENSCHLICHEN ODER TIERISCHEN KÖRPER**
MEDICAL DEVICE FOR INSERTION INTO THE HUMAN OR ANIMAL BODY
DISPOSITIF MÉDICAL DESTINÉ À ÊTRE INTRODUIT DANS LE CORPS D'UN HOMME OU D'UN ANIMAL

(30) Priorität: 21.09.2011 DE 102011113816; 22.09.2011 US 201161537685 P
(43) Veröffentlichungstag der Anmeldung: 30.07.2014
(73) Patentinhaber: MaRVis Medical GmbH, 50226 Frechen (DE)
(72) Erfinder: DÜRING, Klaus, 50226 Frechen (DE)
(74) Vertreter: Schüssler, Andrea
(86) Internationale Anmeldenummer: PCT/EP2012/003963
(87) Internationale Veröffentlichungsnummer: WO 2013/041235

(56) Entgegenhaltungen:
- DE-A1- 4 124 606
- US-A1- 2003 208 142
- US-A1- 2008 312 597
- US-B1- 6 844 492

## Beschreibung

Die vorliegende Erfindung betrifft eine medizinische Vorrichtung zum Einbringen in den menschlichen oder tierischen Körper.

In der EP 1 356 845 D1 ist ein Katheter offenbart. Dieser Katheter weist einen Katheterschaft auf, in dem ein axial durchgehendes, zentrales Lumen angelegt ist. In einer Wandung des Katheterschaftes läuft eine gewendelte Anschlussleitung für eine Ringelektrode, die in einem das distale Ende des Katheters bildenden isolierenden Kopfkörper angeordnet ist. Weiterhin umfasst der Katheter eine in einem Lumen verlaufende Mandrinhülse und einen in der Mandrinhülse verlaufenden Führungsdraht. Der Führungsdraht ist durch eine Schleusenöffnung am distalen Ende des Katheters herausführbar. Proximal vor der Schleusenöffnung ist eine von der Mandrinhülse reversibel durchstoßbare Dichteinheit zur Abdichtung des Lumens vorgesehen. Eine Vorrichtung nach dem einleitenden Teil von Anspruch 1 ist aus DE4124606 bekannt.

Schrittmachersonden werden vaskulär durch Punktion eines Blutgefäßes bis zum Herzen vorgeschoben und dort positioniert. Zur Positionierung wird entweder ein Führungsdraht oder ein Mandrin eingesetzt. In der Regel wird der Führungsdraht in axialer Richtung hin zum distalen Ende eingebracht, so dass der Katheter der Schrittmachersonde über den Führungsdraht geschoben werden kann. Dabei ist das distale Ende des Katheters soweit offen, dass der Führungsdraht aus dem Katheter heraustreten kann. Wird ein Mandrin verwendet, so ist das distale Ende des Katheters verschlossen. Der Mandrin ist mit einer Kugel oder ähnlichem am distalen Ende versehen, so dass das distale Ende der Schrittmachersonde nicht durchstoßen und das Herzgewebe verletzt werden kann. Weiterhin kann die Spitze der Schrittmachersonde mit Röntgenmarkern versehen sein, um eine Kontrolle der Positionierung durch Röntgenbildgebung zu ermöglichen. Bei der Röntgenbildgebung kann kein direktes Abbild des Herzens erzeugt werden. Dies ist nur mit Hilfe eines Kontrastmittels möglich, welches den Blutfluss abbildet. Somit kann also nur mit Stromableitung ins Herz versucht werden, eine geeignete Positionierung für die Schrittmachersonde in der Herzwandung zu finden, so dass diese nicht in nekrotischem Gewebe angeordnet wird. Folglich sind üblicherweise mehrere, häufig 6 bis 8 Positionierungsversuche für eine Schrittmachersonde in der Herzwandung erforderlich, bis eine geeignete Position gefunden worden ist. Bei jedem Positionierungsversuch und anschließendem Wiederherausziehen der Schrittmachersonde wird die Herzwandung leicht verletzt.

Computertomographie (CT) ermöglicht durch die Schichtbildgebung mit Röntgenstrahlen eine Abbildung von Weichgewebe, so dass die Positionierung besser Überprüft werden kann. Hierbei ist die Strahlenbelastung aber relativ hoch.

Magnetresonanztomographie (MRT)-kompatible Herzschrittmacher sind kommerziell erhältlich (z.B. von den Firmen Biotronik und Medtronic). Um die Funktionsfähigkeit des Herzschrittmachers durch die MRT nicht zu beeinträchtigen, wurden magnetisch aufladbare Teile im Herzschrittmacher ausgetauscht und Schaltungen abgeschirmt. Diese MRT-kompatiblen Herzschrittmacher und Schrittmachersonden können aber nicht unter MRT-Führung eingesetzt werden, da dafür entweder ein MRT-kompatibler Führungdraht oder ein MRT-kompatibler Mandrin erforderlich ist. Die heutigen Mandrins aus Metall sind in der MRT wegen Stromleitung und Erhitzung gefährlich. Patienten, die einen MRT-kompatiblen Herzschrittmacher erhalten haben, dürfen erst einige Wochen nach dessen Implantation in eine MRT-Untersuchung gebracht werden.

Die Positionierung von Herzschrittmachersonden würde unter MRT-Bildgebung aufgrund der klaren Darstellung des Herzwandgewebes und der nekrotischen Stellen, in die die Schrittmachersonden nicht gesetzt werden dürfen, da dort keine Herzmuskelaktvität und Stromperzeptivität mehr vorhanden ist, in einem Schritt möglich werden. Damit würde das Herzwandgewebe patientenfreundlich stark geschont und die Dauer der Schrittmachersondenplatzierung erheblich verkürzt werden. Gleiches gilt z.B. für Defibrillatoren, für die gleichermaßen die Sonden gesetzt werden müssen.

Die Brachytherapie ist eine Form der Strahlentherapie, bei der eine Strahlenquelle innerhalb oder in unmittelbarer Nähe des zu bestrahlenden Gebietes im Körper platziert wird. Die Brachytherapie wird häufig als eine wirksame Behandlungsmethode für Krebserkrankungen des Gebärmutterhalses, der Prostata, der Brust und der Haut eingesetzt. Sie kann auch für Tumorbehandlungen an zahlreichen anderen Körperstellen verwendet werden. In der Brachytherapie werden Strahlenquellen, insbesondere Iridium, die an der Spitze eines Einführstabes angeordnet sind, in einen distal geschlossenen Applikator eingebracht, so dass die Strahlenquelle nicht mit dem Tumor selbst in Berührung kommt, aber gezielt zentral in den Tumor positioniert werden kann. Mittels bildgebender Verfahren wird die bestmögliche Platzierung im Tumor bestimmt und erreicht. Für die Bestimmung der für die Therapie erforderlichen Strahlendosen werden mehrfach Positionierungsmessungen, heutzutage üblicherweise unter Röntgenbildgebung, durchgeführt. Die Sichtbarmachung von Tumoren ist in sehr vielen Fällen aber viel besser und präziser erreichbar durch MRT, da die MRT eine viel bessere Weichgewebedarstellung und einen viel höheren Weichteilkontrast bietet.

Aus der WO 2007/000148 A2 geht ein stabförmiger Körper, der zur Ausbildung medizinischer Instrumente, wie zum Beispiel von Kathetern oder Führungsdrähten für Katheter, dient, hervor. Dieser stabförmige Körper besteht aus einem oder mehreren Filamenten und einem nicht-ferromagnetischen Matrixwerkstoff, wobei der Matrixwerkstoff die Filamente umschließt. In dem Matrixwerkstoff ist eine Dotierung aus magnetresonanztomographische Artefakte erzeugenden Partikeln eingebracht.

In der WO 2009/141165 ist ein medizinisches Instrument beschrieben, das in einen menschlichen oder tierischen Körper einführbar ist, wobei das medizinische Instrument einen Instrumentenkörper aufweist. Der Instrumentenkörper weist zumindest einen schlecht elektrisch leitenden stabförmigen Körper auf, der aus einem Matrixwerkstoff und nicht-metallischen Filamenten ausgebildet ist. Dieses medizinische Instrument zeichnet sich dadurch aus, dass der stabförmige Körper mit einem Röntgen-Marker dotiert ist, und das medizinische Instrument einen MRT-Marker aufweist.

Aus der DE 3 417 479 A1 geht ein Venenkatheter mit Führungsmandrin zur EKG-Abnahme hervor. Der Führungsmandrin besteht aus einem elektrisch leitenden Metalldraht mit Kunststoffummantelung.

In der DE 35 26 738 C2 ist eine Vorrichtung zur Lagekontrolle eines zentralvenösen Katheters ofenbart. Hierbei wird ein aus einem Metallstrang und mit Kunststoff ummantelter Mandrin verwendet.

Die DE 41 24 606 A1 beschreibt eine aus einer Metallspirale ausgebildeten Mandrin.

Die DE 10 2005 022 688 A1 betrifft einen Führungsdraht mit einer Ummantelung, die steifer ist als ein darin befindlicher Kern.

Aufgabe der vorliegenden Erfindung ist es, eine medizinische Vorrichtung bereitzustellen, die an einem vorbestimmten Ort im menschlichen Körper anordbar ist.

Eine weitere Aufgabe der vorliegenden Erfindung ist es, eine medizinische Vorrichtung bereitzustellen, deren Biegesteifigkeit veränderbar ist.

Eine weitere Aufgabe der vorliegenden Erfindung ist es, eine medizinische Vorrichtung bereitzustellen, die während des Einbringens bzw. während der Positionierung im menschlichen und/oder tierischen Körper mittels MRT sichtbar gemacht werden kann.

Eine oder mehrere der oben genannten Aufgaben werden durch die in den Patentansprüchen angegebenen Merkmale gelöst.

Eine erfindungsgemäße medizinische Vorrichtung zum Einbringen in den menschlichen und/oder tierischen Körper umfasst eine langgestreckte, rohrförmige Lead-Sonde mit einem proximalen und einem distalen Endbereich, wobei am proximalen Ende der Lead-Sonde eine Öffnung ausgebildet ist, die in ein sich in axialer Richtung erstreckendes Lumen mündet, und das distale Ende der Lead-Sonde verschlossen ist. Weiterhin weist die medizinische Vorrichtung eine langgestreckte Seele auf, wobei die Seele derart ausgebildet ist, dass sie über das Lumen in die Lead-Sonde einbringbar und wieder entfernbar ist, und die Seele aus nicht-metallischen Filamenten und einem Matrixwerkstoff ausgebildet ist.

Unter einer Lead-Sonde wird im Rahmen der vorliegenden Erfindung ein langgestreckter rohrförmiger Körper verstanden, der in einen menschlichen oder tierischen Körper einbringbar und zur Aufnahme einer Seele ausgebildet ist. Eine solche Lead-Sonde ist z.B. Bestandteil einer Schrittmachersonde oder kann als Schlauch oder Hohlnadel, die als Applikator zur Brachytherapie verwendet wird, ausgebildet sein.

Wenn die medizinische Vorrichtung als Schrittmachersonde ausgebildet ist, ist die Lead-Sonde entsprechend als flexibler oder als steifer Schlauch bzw. Katheter ausgebildet.

Wenn die medizinische Vorrichtung zur Verwendung in der Brachytherapie vorgesehen ist, ist die Lead-Sonde entsprechend als Applikator, bspw. als steife Hohlnadel oder als flexibler Kunststoffschlauch zur Brachytherapie ausgebildet.

Unter einer Seele wird im Rahmen der vorliegenden Erfindung ein langgestrecktes stabförmiges Verbundelement verstanden, das in eine Lead-Sonde einführbar ist. Die Seele kann aus nicht-metallischen Filamenten, die von einem Matrixwertstoff umschlossen werden, ausgebildet sein. Optional können Markerpartikel für die Sichtbarmachung der Seele in den Matrixwerkstoff eingebettet werden. Der Matrixwerkstoff ist vorzugsweise ein Kunststoff, wie z.B. Epoxidharz, PEEK, PEBAX, PE, PP, PU, Silikon, Polymilchsäurepolymere. Die Filamente sind bspw. Glasfasern, Keramikfasern, Dacron®, Aramid, Polyaramid, Kevlar®, Dyneema® oder pflanzliche Fasern (z.B. Seide, Sisal, Hanf, etc.).

Aus dem Stand der Technik bekannte Schrittmacher- und Defibrillatorsonden weisen in der Regel einen Führungsdraht oder einen Mandrin auf.

Die Führungsdrähte sind meist mit einer Metallseele versehen und die Mandrins sind in der Regel aus Edelstahl oder einem anderen Metall ausgebildet. Daher sind diese nicht MRTkompatibel.

Mit der erfindungsgemäßen medizinischen Vorrichtung wird ein geschlossenes System mit einer Lead-Sonde bereitgestellt, in dessen Lumen eine als Kunststoffkörper ausgebildete Seele verschieblich angeordnet ist, so dass die medizinische Vorrichtung MRT-kompatibel und -sicher ist.

Der Durchmesser der Seele beträgt vorzugsweise in etwa 70% bis 95 % des Durchmessers des Lumens der Lead-Sonde. Der verbleibende Ringspalt zwischen Seele und Lead-Sonde, in dem Luft angeordnet ist, ist dadurch so klein, dass er bei der Sichtbarmachung mittels MRT keine störenden Artefakte (schwarze Flecken im Bild) erzeugt.

Der Erfinder hat überraschend herausgefunden, dass ein kleiner Luftspalt zwischen der Seele und der Lead-Sonde, d.h. das damit verbundene Restvolumen an Luft in der medizinischen Vorrichtung, die MRT-Bildgebung nicht beeinträchtigt bzw. nicht stört. Es wurden keine darauf zurückzuführenden Artefakte detektiert.

Die Seele weist vorzugsweise eine höhere Biegesteifigkeit als die Lead-Sonde auf. Hierdurch ist die Biegesteifigkeit der Lead-Sonde durch die axiale Position der Seele in der Lead-Sonde stark veränderbar.

Durch das Vorsehen einer als Schrittmachersonde ausgebildeten medizinischen Vorrichtung aus einer flexiblen bzw. weichen Lead-Sonde mit daran angeordneter Elektrode und einer in der Lead-Sonde angeordneten Seele, die eine höhere Biegesteifigkeit aufweist als die Lead-Sonde, ist es möglich, das distale Ende der medizinischen Vorrichtung beim Einbringen und beim Positionieren an die Geometrie der Gefäßwandungen anzupassen und an die Zielposition im Gewebe gerichtet heranzuführen. Die Lead-Sonde lässt sich durch die Seele aufrichten bzw. versteifen, um die Schrittmachersonde gezielt im gesunden Herzwandungsgewebe zu positionieren.

Gleiches gilt auch für eine medizinische Vorrichtung zur Brachytherapie, deren Lead-Sonde die oben beschriebenen Eigenschaften aufweist. In diesem Fall wird die Lead-Sonde gezielt mit Hilfe der Seele in das Tumorgewebe gesteuert.

Bevorzugt weist die Seele einen MRT-Marker auf, wobei sich der MRT-Marker insbesondere über einen Großteil der Länge der Seele erstreckt. Durch den MRT-Marker ist die Position der medizinischen Vorrichtung im menschlichen oder tierischen Körper detektierbar.

Hierdurch ist es möglich, während der Implantation des Schrittmachers oder des Defibrillators unter permanenter Sichtkontrolle in der MRT zu arbeiten. Auf diese Weise kann das vernarbte Gewebe vom gesunden Gewebe unterschieden werden, um gesundes Gewebe zum Einbringen der Schrittmachersonde zu lokalisieren.

Wenn eine Lead-Sonde zur Brachytherapie, die z.B. als steife Hohlnadel ausgebildet ist, eine Seele umfasst, die mit MRT-Markern versehen ist, ist es möglich während der Implantation der Lead-Sonde unter permanenter Sichtkontrolle in der MRT zu arbeiten. Auf diese Weise kann das Tumorgewebe vom gesunden Gewebe unterschieden werden, um das Tumorgewebe für die Einbringung der Strahlungsquelle zu lokalisieren. Dabei kann das Tumorgewebe in einem Körperhohlraum, z.B. Gebärmutterhals, Uterus oder Vagina, einem Körperlumen, wie z.B. Luftröhre oder Speiseröhre oder auch in einem Blutgefäß sitzen. In diesen Fällen wird die Lead-Sonde durch den Körperhohlraum oder -lumen eingeführt. In anderen Fällen, z.B. bei Tumoren in einem Organ wie Leber oder Niere, wird die Lead-Sonde interstitiell durch die Haut und das Gewebe zum Tumor geführt.

Ist die Lead-Sonde im Bereich des Tumorgewebes angeordnet, lässt sich die Seele entfernen und an deren Stelle wird eine Strahlenquelle in die Lead-Sonde eingebracht. Auf diese Weise lässt sich die Strahlenquelle in unmittelbarer Nachbarschaft zum bzw. direkt im Tumorgewebe anordnen, so dass möglichst wenig gesundes Gewebe bei der Strahlentherapie beschädigt wird.

Bei der Brachytherapie ist es vorteilhaft, dass durch ein Herausziehen der Seele aus der Lead-Sonde das Gewebe unter Magnetresonanztomographie analysiert werden kann, ohne dass ein MRT-Artefakt erzeugt wird, welches das Zielgewebe lokal überlagern und damit die Genauigkeit der Zielsteuerung beeinträchtigen könnte. Durch mehrfaches stückweises Herausziehen und Hineinschieben der Seele können sehr einfach abwechselnd MRT-Bilder zur Visualisierung des Applikators und des Tumors aufgenommen werden. Ein fest angebrachter MRT-Marker bietet diesen Vorteil nicht.

Sollte das Luftvolumen in der Lead-Sonde, wenn die Seele mit MRT-Marker entfernt worden ist, ein das Gewebe überlagerndes, störendes Luftartefakt erzeugen, so kann eine andere Seele ohne MRT-Marker eingeführt werden, die das Luftartefakt beseitigt. Bei Verwendung einer Lead-Sonde, die nur aus Kunststoff besteht und kein eigenes Artefakt erzeugt (außer dem Verdränungsartefakt), kann durch den Wechsel zwischen einer Seele mit MRT-Marker und einer Seele ohne MRT-Marker je nach Bedarf ein eindeutiges Artefakt im MRT für die präzise Lokalisierung der medizinischen Vorrichtung erzeugt werden oder ein (annähernd) artefaktfreies Abbild an der Position der medizinischen Vorrichtung.

Weiterhin kann auch vorgesehen sein, dass die medizinische Vorrichtung eine Seele aufweist, die sowohl eine höhere Biegesteifigkeit als die Lead-Sonde als auch MRT-Marker aufweist.

Auf diese Weise wird eine Kombination der oben beschriebenen Vorteile erreicht.

Bspw kann dann das Einbringen einer Schrittmachersonde überwacht werden, wenn die Schrittmachersonde selbst kein oder kein reproduzierbares sicheres MRT-Artefakt erzeugt. Anschließend lässt sich das distale Ende der Schrittmachersonde im Bereich des gesunden Gewebes durch die Seele aufrichten bzw. versteifen und somit exakt positionieren.

Eine medizinische Vorrichtung zur Brachytherapie ist beim Einbringen und beim Positionieren durch die variable Biegesteifigkeit an die Geometrie der Hohlraumwandungen anpassbar und kann durch die MRT-Marker optisch überwacht werden, bis sie im Tumorgewebe angeordnet ist. Ist das distale Ende der medizinischen Vorrichtung dann in der Nähe des Tumorgewebes oder im Tumorgewebe angeordnet, lässt sich dieses durch die Seele aufrichten bzw. versteifen und auf diese Weise exakt positionieren.

Die Erfindung wird nachfolgend beispielhaft näher anhand der in den Zeichnungen dargestellten Ausführungsbeispiele erläutert. Die Zeichnungen zeigen in:

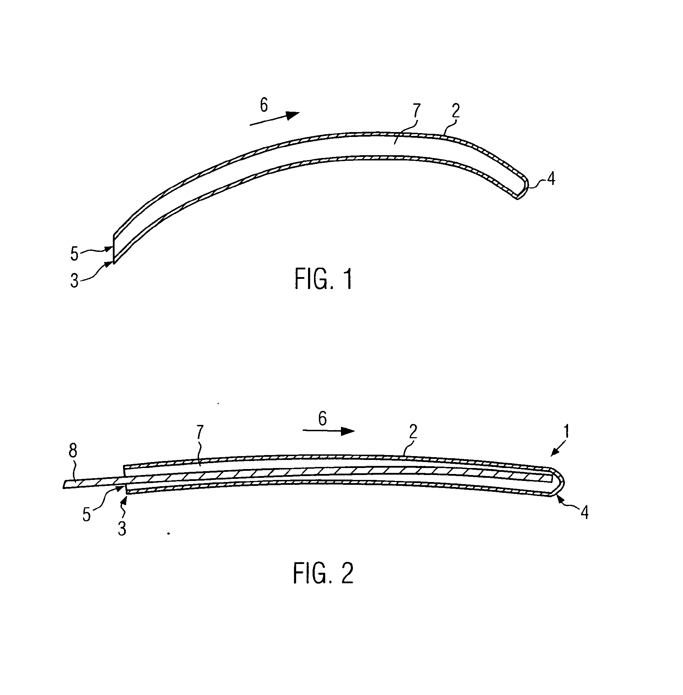

- Figur 1: eine Lead-Sonde in einer seitlich geschnittenen Ansicht,
- Figur 2: eine Lead-Sonde mit darin angeordneter Seele in einer seitlich geschnittenen Ansicht,
- Figur 3: eine Seele im Querschnitt aus nicht-metallischen Filamenten, die von einem Matrixwerkstoff umschlossen sind,
- Figur 4: einen Querschnitt der in Figur 3 gezeigten Seele mit einer Hüllmatrix,
- Figur 5: eine Seele im Querschnitt aus mehreren in eine Hüllmatrix eingebetteten Verbundelementen,
- Figur 6: eine Seele im Querschnitt ausgebildet aus einem in eine Hüllmatrix eingebetteten Verbundelement,
- Figur 7: Bilder einer Testanordnung, die mittels Magnetresonanztomographie erstellt worden sind,
- Figur 8: Bilder von einer Testanordnung, die mit mittels Magnetresonanztomographie erstellt worden sind,

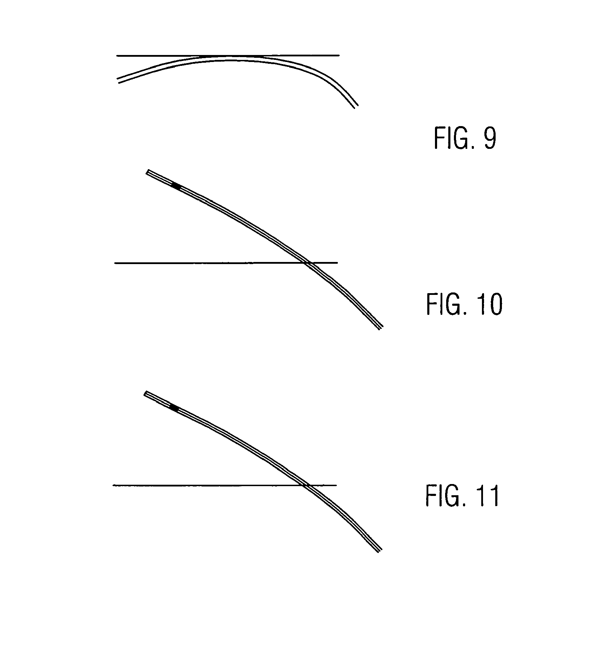

- Figur 9: eine Lead-Sonde ohne Seele in einer Seitenansicht,
- Figur 10: eine Lead-Sonde mit einem Edelstahlmandrin gemäß dem Stand der Technik in einer Seitenansicht, und
- Figur 11: eine Lead-Sonde mit einer erfindungsgemäßen Seele.

Im Folgenden werden bevorzugte Ausführungsformen der vorliegenden Erfindung beschrieben.

Eine erfindungsgemäße medizinische Vorrichtung 1 zum Einbringen in den menschlichen und/oder tierischen Körper umfasst eine langgestreckte, rohrförmige Lead-Sonde 2 mit einem proximalen 3 und einem distalen Endbereich 4, wobei am proximalen Ende 3 der Lead-Sonde 2 eine Öffnung 5 ausgebildet ist, die in ein sich in axialer Richtung 6 erstreckendes Lumen 7 mündet, und das distale Ende der Lead-Sonde 2 verschlossen ist. Weiterhin weist die medizinische Vorrichtung 1 eine langgestreckte Seele 8 auf, wobei die Seele 8 derart ausgebildet ist, dass sie über das Lumen 7 in die Lead-Sonde 2 einbringbar und wieder entfernbar ist, und die Seele 8 aus nicht-metallischen Filamenten 9 und einem Matrixwerkstoff 10 ausgebildet ist.

Im Folgenden werden die verschiedenen Ausführungsformen einer erfindungsgemäßen Seele beschrieben.

Die Seele 8 weist zumindest ein langgestrecktes stabförmiges Verbundelement 13 auf, das aus Glasfaserbündeln 12 bzw. Glasfaser-Rovings ausgebildet ist, die in einen Matrixwerkstoff, wie z.B. Epoxidharz, eingebettet sind. Durch das Vorsehen der Glasfasern 12 wird eine Seele 8 mit hoher Biege- und Torsionssteifigkeit bereitgestellt.

Die Fasern erstrecken sich vorzugsweise über einen Großteil der Länge der Seele 8 und insbesondere über die gesamte Länge der Seele 8. Sie verlaufen in der Seele 8 vorzugsweise im wesentlichen parallel.

Weiterhin kann die Seele zusätzlich zu den Glasfasern 12 auch Aramidfasern 11 enthalten. Die Aramidfasern 11 sorgen für eine hohe Reißfestigkeit der Seele 8.

Je höher der Anteil der Glasfaser 12 in der Seele 8 ist, desto höher ist die Steifigkeit bzw. Biegesteifigkeit der Seele 8.

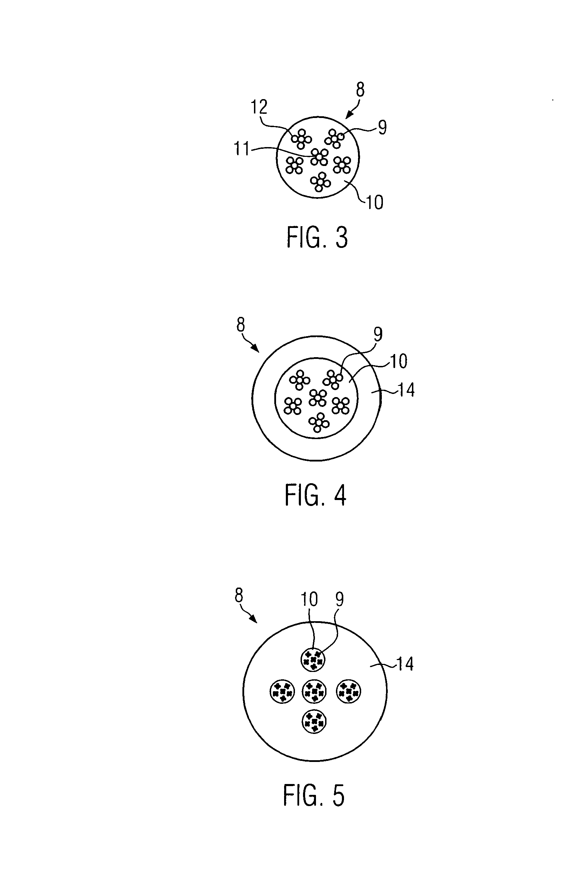

Eine Seele 8 kann beispielsweise aus einem zentralen 11Tex Aramidfaser-Roving 11 und fünf den zentralen Aramidfaser-Roving 11 konzentrisch umgebende und gleich beabstandet voneinander angeordnete, 33Tex Glasfaser-Rovings 12 ausgebildet sein (Figur 3). Anstelle von Rovings ist es auch möglich Garne (yarns) zu verwenden, wobei Rovings bevorzugt werden, da bei kleineren Durchmessern höhere Festigkeiten erzielbar sind. Eine derart ausgebildete Seele weist einen Außendurchmesser von 0,35 mm bis 0,38 mm auf.

Alternativ können anstelle der 11Tex Aramidfaser-Rovings auch 6.1Tex Aramidfaser-Rovings verwendet werden. Eine derart ausgebildete Seele weist einen Außendurchmesser von ca. 0,34 mm bis 0,36 mm auf.

In einer anderen Ausführungsform kann anstelle des 11Tex Aramidfaser-Rovings auch ein zentraler 22Tex Aramidfaser-Roving verwendet werden, der konzentrisch gleich beabstandet voneinander von sieben 33Tex Glasfaser-Rovings umgeben ist. Eine derart ausgebildete Seele weist einen Außendurchmesser von 0,42 bis 0,45 mm auf.

In einer weiteren Ausführungsform kann die Seele 8 bei einem Durchmesser der Seele von in etwa 0,35 mm bis 0,38 mm ca. 200Tex Glasfaser enthalten, die in Form von drei 66Tex Glasfaser-Rovings eingebracht werden.

Durch mehr Epoxidharz und eine höhere Anzahl von Fasern lassen sich selbstverständlich auch Verbundelemente 13 mit größerem Durchmesser und höherer Festigkeit herstellen.

Das Verbundelement 13 kann gemäß den in der WO 2009/141165 oder WO 2007/000148 A2 beschriebenen stabförmigen Körpern aufgebaut sein, auf die hiermit voll inhaltlich Bezug genommen wird.

Die Oberfläche der Seele kann nach außen abstehende Glasfasern oder andere nicht-metallischen Filamente enthalten, welche die Produktsicherheit beeinträchtigen können. Um eine glatte Oberfläche ohne abstehende Filamente zu erhalten, kann die Seele mit einem etwas größeren Durchmesser als dem Zieldurchmesser hergestellt und anschließend durch Abschleifen auf den Zieldurchmesser gebracht werden. Durch das Abschleifen der Oberfläche auf den Zieldurchmesser ergibt sich auch eine höhere Flexibilität in der Produktion, da mit Standard-Glasfaser- und Aramid-Rovings nur bestimmte Geometrien realisiert werden können und bestimmte resultierende Außendurchmesser erzielbar sind. Handelsübliche Rovings sind nur in bestimmten Durchmessern erhältlich. Wenn ohne Schleifen ein stabförmiger Körper mit einem Durchmesser zwischen den Standardgrößen zweier Rovings hergestellt wird, dann weist er eine äußere Schicht auf, die ausschließlich aus Matrixwerkstoff besteht und nicht durch nicht-metallischen Filamente verstärkt ist. Dies ist nicht optimal, da die Festigkeit der äußeren Schicht besonders stark die Steifigkeit des gesamten stabförmigen Körper beeinflusst. Der Durchmesser eines stabförmigen Körpers wirkt sich mit der vierten Potenz auf dessen Steifigkeit aus.

Durch das Abschleifen können somit stabförmige Körper mit beliebig feinen Abstufungen und über die gesamte Querschnitsfläche mit gleichmäßig verteilten nicht-metallischen Filamenten hergestellt werden. Das Verhältnis der Querschnittsflächen von nicht-metallischen Filamenten zum Matrixmaterial, insbesondere zum Epoxidharz, beträgt vorzugsweise 60:40, wobei diese Werte um ±10 variieren können.

Um den Durchmesser der Seele 8 zu erhöhen bzw. um den Luftspalt zwischen der Außenwandung der Seele und der Innenwandung der Lead-Sonde 2 zu reduzieren, kann vorgesehen sein, die oben beschriebenen Seelen mit einer Hüllmatrix 14 zu umhüllen (Figur 4). Die Hüllmatrix ist bspw. aus PU ausgebildet.

Eine Seele 8 kann auch derart ausgebildet sein, dass mehrere der oben beschriebenen Verbundelemente 13 in eine Hüllmatrix 14 eingebettet sind (Figur 5). Optional können Markerpartikel für die Sichtbarmachung der Seele 8 mittels MRT in den Matrixwerkstoff und/oder in der Hüllmatrix eingebettet sein. In den Matrixwerkstoff 10 aus Epoxidharz werden vorzugsweise Eisenmikropartikel als MRT-Marker eingebettet.

Die Untergrenze für die Größe der Eisenmikropartikel beträgt zumindest 1 µm bzw. 10 µm bzw. 20 µm bzw. 30 µm bzw. 40 µm. Es gilt grundsätzlich, dass je größer die Partikel sind, desto stärker sind die hierdurch erzeugten Artefakte und desto deutlicher sind sie unter MRT sichtbar. Die Obergrenze der Größe der Eisenmikropartikel beträgt kleiner 150 µm bzw. 100 µm bzw. 80 µm bzw. 70 µm bzw. 60 µm. Bei großen Partikelgrößen von etwa 100 µm und mehr ist die Verarbeitung schwieriger und es ist nicht einfach, sie gleichmäßig in dem Matrixwerkstoff zu verteilen. Vorzugsweise liegt die Größe der Mikropartikel in einem Bereich zwischen 20 µm und 70 µm und besonders bevorzugt zwischen 40 und 60 µm

Zusätzlich zu den im Matrixwerkstoff und/oder in der Hüllmatrix enthaltenen MRT-Markerpartikeln kann auch ein separater MRT-Spitzenmarker am distalen Endbereich der Seele aufgebracht werden, der ein breiteres Artefakt, z.B. mit der zwei- bis dreifachen Breite des Artefakts über die gesamte Länge der Seele, im MRT-Bild erzeugt. Mit solch einem Spitzenmarker kann die Spitze der Seele und damit das distale Ende der medizinischen Vorrichtung eindeutig im MRT-Bild identifiziert werden. Ist der Spitzenmarker nicht sichtbar im MRT-Bild, so befindet sich die Spitze nicht in der visualisierten Schicht der MR-Aufnahme und die Schichteinstellung muss nachgestellt werden.

Der MRT-Spitzenmarker wird bspw. durch Auftragen einer selbsthärtenden Polymerlösung aufgetragen, die MRT-Markerpartikel enthält. Nach dem Aushärten bildet die Polymerlösung eine Schicht, die sich vorzugsweise über einen Längsbereich von einigen µm bis zu einigen mm erstreckt. Die Schicht kann alleine an einer Stirnfläche des stabförmigen Körpers aufgebracht sein, so dass die Längserstreckung der Schichtdicke entspricht. Die Schicht kann jedoch auch auf de Mantelfläche des stabförmigen Körpers aufgetragen sein, wobei dann die Längserstreckung vorzugsweise nicht länger als 5 mm und insbesondere nicht länger als 3 mm ist. Die Polymerlösung kann z.B. aus PEBAX bestehen. Als MRT-Markerpartikel können alle hierin offenbarten MR-Marker verwendet werden, wobei Eisen-Markerpartikel bevorzugt werden.

Der Spitzenmarker kann zusätzlich zu MRT-Marker der Seele vorgesehen sein, so dass er im Bereich der Spitze ein stärkeres Artefakt als im übrigen Bereich der Seele bei einem MRT-Bildgebungsverfahren erzeugt.

Die abwechselnde Verwendung von nicht-markierten Seelen, von nur kontinuierlich markierten Seelen und von kontinuierlich und spitzenmarkierten Seelen ermöglicht die höchstmögliche Flexibilität zur exakten und optimalen Positionierung der medizinischen Vorrichtung am oder im Zielgewebe unter permanenter MRT-Sichtkontrolle.

Die Seele 8 weist im Querschnitt vorzugsweise eine annähernde Kreisform auf, da das Lumen 7 der Lead-Sonde 2 ebenfalls kreisförmig ist. Der Innendurchmesser des Lumens 7 der Lead-Sonde 2 ist vorzugsweise nur etwas größer als der Außendurchmesser der Seele 8, so dass diese annähernd reibungsfrei durch die Lead-Sonde 2 verschiebbar ist und lediglich ein minimaler Luftspalt zwischen der Außenfläche der Seele 8 und der Innenfläche der Lead-Sonde 2 ausgebildet wird.

Der Durchmesser der Seele 8 beträgt in etwa 60 % - 98 % bzw. 70 % - 95 % bzw. 80 %-90 % des Durchmessers des Lumens 7 der Lead-Sonde 2. Hierbei ist wichtig, dass die Oberfläche der Seele 8 und insbesondere deren distale Spitze einfach durch das Lumen 7 der Lead-Sonde 2 hindurchgleiten. Dafür kann die distale Spitze gemäß den aus dem Stand der Technik bekannten Verfahren verrundet werden. Weiterhin weist die Seele 8 vom proximalen 3 bis zum distalen Ende 4 eine weitestgehend homogene Steifigkeit auf. In einer alternativen Ausführungsform kann das distale Ende der Seele 8 eine abnehmende Steifigkeit aufweisen. Diese kann durch Abschleifen des Seelenkörpers erreicht werden, wobei eine Reduzierung des Außendurchmessers in diesem Abschnitt resultiert.

Im Folgenden werden verschiedene Ausführungsformen der medizinischen Vorrichtung 1 zum Einbringen in den menschlichen oder tierischen Körper umfassend eine Lead-Sonde 2 und eine Seele 8 beschrieben.

Gemäß einem ersten Ausführungsbeispiel der vorliegenden Erfindung ist die medizinische Vorrichtung 1 als Schrittmachersonde ausgebildet, die mit einer entsprechenden Elektrode (nicht dargestellt) für Herzschrittmacher oder Defibrillatoren versehen ist, wobei die Lead-Sonde 2 einen schlauchförmigen Katheter bildet, an dessen distalen Ende 4 eine entsprechende Elektrode angebracht ist. Eine derartige Lead-Sonde 2 ist weich bzw. flexibel ausgebildet. Die in der Lead-Sonde 2 anordbare Seele 8 ist derart ausgebildet, dass sie eine höhere Biegesteifigkeit als die Lead-Sonde 2 aufweist. Hierfür wird eine Seele 8 verwendet, die einen maximalen Anteil Glasfaser-Bündel aufweist, da diese eine hohe Biegesteifigkeit gewährleisten. Vorzugsweise weist die Seele auch Aramidfasern auf, die eine hohe Reißfestigkeit sicher stellen.

Hierdurch ist es möglich, das distale Ende 4 der medizinischen Vorrichtung 1 beim Einbringen und beim Positionieren an die Geometrie der Gefäßwandungen anzupassen und an gesundes Herzwandungsgewebe heranzuführen.

Da der Durchmesser des Lumens 7 einer solchen Lead-Sonde 2 typischerweise etwa 0,4 mm beträgt, ist der Durchmesser der Seele 8 entsprechend anzupassen. Eine solche Seele 8 besteht vorzugsweise nur aus dem stabförmigen Verbundelement 13 ohne Hüllmatrix 14 (siehe Figur 3).

Die Biegesteifigkeit der medizinischen Vorrichtung 1 lässt sich durch Verschieben der Seele 8 in axialer Richtung bzgl. der Lead-Sonde 2 verändern, um die Schrittmachersonde gezielt im gesunden Herzwandungsgewebe zu positionieren.

Gemäß einer weiteren Ausführungsform der medizinischen Vorrichtung entspricht die Biegesteifigkeit der Seele 8 in etwa der Biegesteifigkeit der Lead-Sonde 2 oder sie ist auch geringer als die Biegesteifigkeit der Lead-Sonde 2. Die Seele 8 ist gemäß diesem Ausführungsbeispiel mit MRT-Markern versehen, wobei sich die MRT-Marker insbesondere über einen Großteil der Länge der Seele 8 oder über die gesamte Länge der Seele 8 erstrecken. Durch die MRT-Marker ist die Position der medizinischen Vorrichtung 1 im menschlichen oder tierischen Körper detektierbar. Die Seele 8 kann Glasfasern aufweisen. Zusätzlich zu den Glasfasern oder auch alternativ zu den Glasfasern kann die Seele Aramidfasern aufweisen, die der Seele eine hohe Reißfestigkeit verleihen.

Hierdurch ist es möglich, während der Implantation der Schrittmacher- oder Defibrillatorsonden in der MRT unter permanenter Sichtkontrolle zu arbeiten. Auf diese Weise kann die Lead-Sonde 2 gezielt und sicher in gesundes Gewebe in der Herzwandung platziert werden, so dass eine Fehlplatzierung in vernarbtem Gewebe und eine daraus notwendig werdende Repositionierung vermieden wird. Somit kann die medizinische Vorrichtung 1 in der Regel in einem Arbeitsgang korrekt positioniert werden.

Gemäß einer weiteren Ausführungsform der vorliegenden Erfindung weist die Seele 8 sowohl eine höhere Biegesteifigkeit als die Lead-Sonde 2 als auch MRT-Marker auf.

Hieraus ergibt sich eine Kombination der oben beschriebenen Vorteile. Es ist somit möglich, während der Implantation der Schrittmacher- oder Defibrillatorsonde unter permanenter Sichtkontrolle in der MRT zu arbeiten und gleichzeitig entsprechend der Biegungen der Gefäßwandungen die Biegesteifigkeit der Lead-Sonde 2 zu verändern. Auf diese Weise ist es möglich, den Biegungen der Blutgefäße zu folgen und schließlich auch das gesunde Herzwandungsgewebe zu detektieren. Das distale Ende der Lead-Sonde 2 kann dann versteift werden, um die Schrittmachersonde gezielt im gesunden Herzwandungsgewebe anzuordnen.

Eine steife Seele 8 erlaubt die Verwendung einer sehr flexiblen Lead-Sonde 2. Dies ist insbesondere von Vorteil, wenn die Lead-Sonde 2 auf Dauer im menschlichen Körper implantiert wird. Je flexibler die Lead-Sonde 2 ist, desto besser passt sie sich der Körper- bzw. Gefäßgeometrie an und vollzieht etwaige Körperbewegungen mit. Die Seele weist vorzugsweise Glasfasern und Aramidfasern auf.

Gemäß weiteren Ausführungsformen der vorliegenden Erfindung ist die medizinische Vorrichtung 1 zur Brachytherapie ausgebildet. Die Lead-Sonde 2 ist dann vorzugsweise entweder als flexibler Schlauch oder als steife Hohlnadel ausgebildet.

Eine steife Hohlnadel ist z.B. bei Tumoren in einem Organ, wie der Leber oder der Niere vorgesehen. Hierbei wird die Lead-Sonde interstitiell durch die Haut und das Gewebe zum Tumor geführt.

Eine flexibler Schlauch ist insbesondere dann vorgesehen, wenn das Tumorgewebe sich in einem Körperhohlraum, z.B. Gebärmutterhals, Uterus oder Vagina oder einem Körperlumen wie z.B. Luft- oder Speiseröhre oder auch in einem Blutgefäß befindet.

Dadurch, dass unter permanenter Sichtkontrolle in der MRT gearbeitet werden kann, wird das Einbringen erheblich erleichtert und das Tumorgewebe kann lokalisiert werden.

Gemäß einer ersten Ausführungsform einer medizinischen Vorrichtung zur Brachytherapie ist die Lead-Sonde als steife Hohlnadel ausgebildet. Weiterhin ist eine Seele vorgesehen, die mit MRT-Markern versehen ist. Hierdurch ist es möglich, während der Implantation der Lead-Sonde unter permanenter Sichtkontrolle in der MRT zu arbeiten. Auf diese Weise kann das Tumorgewebe vom gesunden Gewebe unterschieden werden, um das Tumorgewebe für die Einbringung der Strahlungsquelle zu lokalisieren.

Hierbei spielt die Steifigkeit der Seele eine untergeordnete Rolle. Es ist jedoch vorteilhaft, wenn die Seele Aramidfasern aufweist, die eine hohe Reißfestigkeit gewährleisten, so dass auch bei starker Zugbelastung in der Handhabung kein Reißen der Seele auftritt.

Ist die Lead-Sonde im Bereich des Tumorgewebes angeordnet, lässt sich die Seele entfernen und an deren Stelle wird eine Strahlenquelle in die Lead-Sonde eingebracht. Die Strahlenquelle ist vorzugsweise aus Iridium ausgebildet. Somit ist es mit der erfindungsgemäßen medizinischen Vorrichtung möglich, die Strahlenquelle in unmittelbarer Nachbarschaft bzw. direkt im Tumorgewebe anzuordnen, so dass möglichst wenig gesundes Gewebe bei der Strahlentherapie beschädigt wird.

Die Lead-Sonde einer medizinischen Vorrichtung zur Brachytherapie weist ein Lumen mit einem Durchmesser von ca. 0,8 mm bis 1,5 mm auf. Der Durchmesser der Seele ist entsprechend anzupassen.

Die Seele kann bei einem solchen Durchmesser aus einem oder mehreren stabförmigen Verbundelementen ausgebildet sein, die in eine Hüllmatrix eingebettet sein können.

Gemäß einer zweiten Ausführungsform einer medizinischen Vorrichtung zur Brachytherapie ist die Lead-Sonde als flexibler Schlauch ausgebildet. Sofern nichts anderes beschrieben ist, weist die medizinische Vorrichtung die Merkmale der ersten Ausführungsform zur Brachytherapie auf.

Die in der Lead-Sonde anordbare Seele ist hierbei derart ausgebildet, dass sie eine niedrigere, eine gleichartige oder eine höhere Biegesteifigkeit als die Lead-Sonde aufweist. Hierfür wird eine Seele 8 verwendet, die einen niedrigeren oder einen höheren Anteil an Glasfaser-Bündeln aufweist, wobei ein höherer Anteil an Glasfaser-Bündeln zu einer höheren Biegesteifigkeit führt. Vorzugsweise weist die Seele 8 auch Aramidfasern auf, die ihr eine hohe Reißfestigkeit verleihen.

Durch die veränderbare Biegesteifigkeit der medizinischen Vorrichtung ist diese beim Einbringen und Positionieren an die Geometrie der Hohlräume anpassbar.

Erfindungsgemäß sind beliebige Kombinationen aus flexiblen bis harten Lead-Sonden mit Seelen, die eine höhere, eine gleiche oder eine geringere Biegesteifigkeit als die Leadsonde aufweisen, und die Markerpartikel für die MRT- oder Röntgenvisualisierung oder keine Markerpartikel aufweisen, vorstellbar. Hierbei wird auf die Figuren 3 bis 6 verwiesen, die beispielhaft entsprechende Seelen für den jeweiligen Anwendungsfall offenbaren.

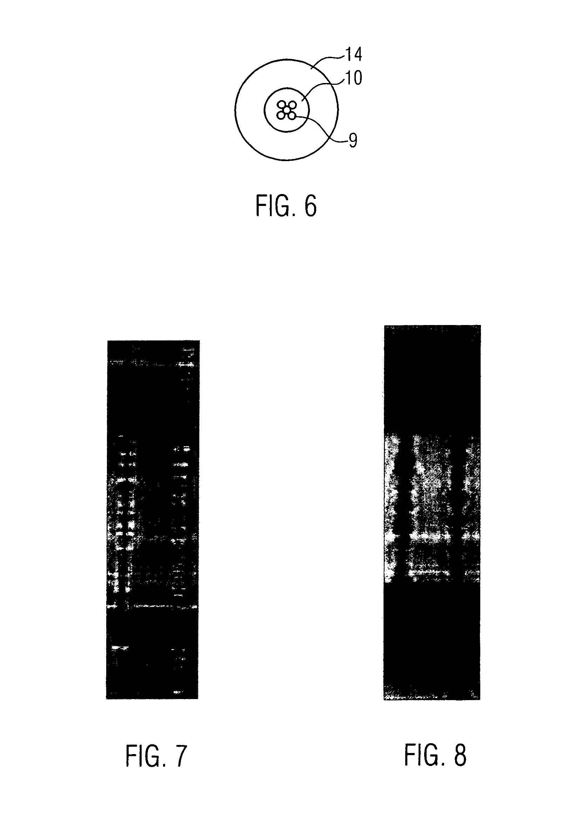

Muss die Seele der medizinischen Vorrichtung keine Steifigkeit verleihen, dann kann sie lediglich ein einziges Verbundelement umfassen (Fig. 6). Das Verbundelement ist von einer Hüllmatrix umgeben, um so an den Innendurchmesser der Lead-Sonde angepasst zu sein. Das Verbundelement ist vorzugsweise konzentrisch in der Seele im Bereich ihrer Längsachse angeordnet, wenn es MRT-Marker enthält, so dass sich die MRT-Marker in einem engen Bereich um die Längsachse der Seele befinden. Bei der Bildgebung erzeugen die MRT-Marker Artefakte, die sich bei der Verwendung herkömmlicher MRT-Sequenzen für die Bilderzeugung über den Bereich des Objektes selbst hinaus erstrecken. Bei dieser Ausführungsform, bei der die MRT-Marker im Zentrum der Seele konzentriert sind, sind die Artefakte so klein wie physikalisch möglich, da der Abstand zu den umgebenden Wassermolekülen, die in der MRT abgebildet werden, so groß wie möglich ist. Hierdurch ist die Seele einerseits sehr gut und lokal begrenzt erkennbar, aber so stark begrenzt, dass die Darstellung des angrenzenden Gewebes nicht beeinträchtigt wird. Durch die Konzentration der Markerpartikel im Zentrum der Seele sowie den durch den luftgefüllten Ringspalt und die Lead-Sonden-Wand vorhandenen Abstand zu den umgebenden Wassermolekülen im Körper wird ein Artefakt erhalten, das so begrenzt wie möglich und damit für die zielgenaue Ansteuerung des Tumors optimiert ist.

Bei Verwendung mehrerer Verbundelemente kann es auch zweckmäßig sein, aus diesem Grund lediglich das zentrale Verbundelement mit MRT-Markern zu dotieren.

Bezüglich der Dotierung mit MRT-Markern wird auf die entsprechenden Textstellen der EP 101 878 63 verwiesen, auf die hiermit vollinhaltlich Bezug genommen wird. Bei der Dotierung mit MRT-Markern sind die Ziele a) ein starkes und b) ein örtlich begrenztes und scharfes Artefakt zu haben. Je stärker das Artefakt ist, desto breiter wird es. Vorzugsweise ist das Artefakt in paralleler Richtung zum Hauptmagnetfeld stark genug und in orthogonaler Richtung nicht zu breit, wobei diese entsprechend ausbalanciert sein sollten.

Die folgenden Effekte beeinflussen die Intensität und die Breite des Artefakts der medizinischen Vorrichtung umfassend eine Lead-Sonde und eine Seele:
- Je höher die Konzentration der MRT-Marker ist, desto stärker und breiter ist das Artefakt.
- Je größer die Partikel sind, desto besser ist die Balance zwischen Bildgebung in paralleler und orthogonaler Richtung. Allerdings kann die Partikelverteilung bei zu großen Partikeln ungleichmäßig werden, so dass kein gleichmäßiges Artefakt mehr erhalten wird. Die Größe der Partikel ist durch den Herstellungsprozess der Verbundelemente (stabförmige Körper) und/oder der medizinischen Vorrichtung begrenzt. Zu große Partikel können nicht mehr durch die Herstellungswerkzeuge passieren. Partikelgrößen im Bereich zwischen 1 µm und 150 µm sind am geeignetsten. Vorzugsweise haben die Partikel eine Größe von zumindest 1 µm, 2 µm, 5 µm, 10 µm, 20 µm, 30 µm, 40 µm oder 50 µm. Gute Ergebnisse werden auch mit Partikeln erzielt, die mit einem Sieb mit einer Maschenweite von 150 µm gesiebt wurden. Ein Sieb mit einer Maschenweite von ungefähr 80 µm bis 130 µm ist ebenfalls geeignet. Besonders bevorzugt sind Eisenpartikel mit einer Größe von 20 bis 100 µm, noch bevorzugter 30 bis 80 µm und am meisten bevorzugt 40 bis 65 µm.
- Mehrere dotierte Verbundelemente liefern ein stärkeres Artefakt als ein einzelnes Verbundelement mit der gleichen oder sogar einer höheren Menge an Markerpartikeln.

Die Größe und insbesondere die Breite des Spitzenmarker-Artefakts ist abhängig von der absoluten aufgetragenen Menge des MRT-Markers.

Der Abstand der Markerpartikel oder der Verbundelemente von umgebenden Wassermolekülen beeinflusst die Schärfe und Intensität des Artefaktes. Je größer der Abstand zwischen den Markerpartikeln und den umgebenden Wassermolekülen ist, desto schwächer ist der Einfluss des magnetischen Feldes auf diese Wassermoleküle. Mit anderen Worten, je größer der Durchmesser der medizinischen Vorrichtung im Verhältnis zum Durchmesser des konzentrisch angeordneten, mit MRT-Markerpartikeln dotierten Verbundelementes 13 ist, desto begrentzer ist das erzeugte Artefakt und umso schärfer ist die resultierende Abbildung der medizinischen Vorrichtung im MRT-Bild.

Diese Effekte können in verschiedenen Kombinationen genutzt werden, um das oben genannte Ziel eines starken und scharfen Artefakts zu erreichen.

Eine Möglichkeit hierfür ist, die Seele mit einem zentralen Verbundelement auszubilden bzw. das oder die Verbundelemente nahe am Zentrum der medizinischen Vorrichtung anzuordnen. Hierdurch wird nur eine vergleichsweise kleine Schicht der umgebenen Wassermoleküle von den Eisenpartikeln im Verbundelement beeinflusst im Vergleich zu einer dickeren Schicht, wenn die Markerpartikel in peripheren Verbundelementen nahe des äußeren Randes der Seele oder gar auf der Oberfläche der medizinischen Vorrichtung angeordnet sind. Je dicker die Schicht der beeinflussten Wassermoleküle ist, desto breiter ist das daraus resultierende Artefakt und Abbild der medizinischen Vorrichtung, welches dann ein größeres Vielfaches ihres eigentlichen Durchmessers sein kann. Im Falle einer medizinischen Vorrichtung, bei der die Seele ein zentrales Verbundelement aufweist, welches die MRT-Markerpartikel beinhaltet, wird ein ausreichend starkes und scharfes Artefakt erhalten, so dass das Abbild nur einen geringfügig größeren Durchmesser aufweist, als der tatsächliche Durchmesser der medizinischen Vorrichtung ist.

Dieser Aufbau der medizinischen Vorrichtung basiert auf dem Einfluss des magnetischen Feldes, verursacht durch die Markerpartikel, auf die Protonen in den Wassermolekülen, die in unmittelbarer Nähe der medizinischen Vorrichtung vorliegen.

Eine andere Möglichkeit, ein stark begrenztes bzw. lokal begrenztes und scharfes Artefakt zu erhalten ist die Einstellung der MR-Sequenz. Wenn die Relaxationsechos der Protonen in einer aus einem weichen Polymer ausgebildeten Hüllmatrix und nicht die in den umgebenden Wassermolekülen detektiert werden, ist es möglich ein sehr scharfes Bild, welches fast nur auf den tatsächlichen Durchmesser der Seele begrenzt ist, zu erhalten. Dies ist bevorzugt wenn es erforderlich ist, das distale Ende einer medizinischen Vorrichtung auf einem schmalen Zielbereich z.B. in vom Krebs befallenen Bereichen zu positionieren. Harte Polymermaterialien wie z.B. Epoxidharze oder Polyurethan oder Thermoplastische Elastomere ausgebildet aus SEBS enthalten eine Vielzahl von Protonen aber die Relaxationszeiten für diese Protonen sind in harten Polymeren, aufgrund der Steifigkeit des Materials, viel zu kurz, um diese mit zurzeit verfügbaren MR-Sequenzen und Scannern zu detektieren. Anstelle dieser relativ harten Polymermaterialien können weichere Polymermaterialien als Hüllmatrix für die Seele verwendet werden. Bei weicheren Polymeren sind die Protonen etwas flexibler und haben längere Relaxationszeiten. Demnach können sie mit zurzeit verfügbaren MR-Scannern und MR-Sequenzsoftware detektiert werden. Beispielsweise PVC oder Gummi sind geeignet für diesen Zweck. Gummimaterialien stellen eine hohe Stabilität bereit aufgrund ihrer quervernetzten Polymerketten, aber die Protonen von Gummimaterialien haben immer noch eine sehr lange Relaxationszeit.

Im Rahmen der Erfindung ist es jedoch auch möglich, die weiteren Verbundelemente mit Markern zu dotieren, insbesondere auch mit anderen Typen von MRT-Markern oder Markern, die bei einer Röntgenbildgebung sichtbar sind. Außerdem kann über die Art und die Konzentration der MRT-Marker die Artefaktintensität und - breite gesteuert werden.

Es kann auch zweckmäßig sein, in die Hüllmatrix, die insbesondere aus PU ausgebildet ist, Röntgen-Marker vorzusehen. Ein geeignetes Material für Röntgen-Marker ist Bariumsulfat. Ein anderes geeignetes Material sind Wolfram-Mikropartikel, vorzugsweise in einer Größe von 1 µm bis 100 µm bzw. 1 µm bis 50 µm bzw. 1 µm bis 15 µm oder Wolfram-Nanopartikel vorzugsweise in einer Größe von 1 nm bis 1000 nm bzw. 10 nm bis 100 nm bzw. 40 nm bis 60 nm.

Nach einem weiteren Aspekt der vorliegenden Erfindung sind die medizinischen Vorrichtungen sowohl mit MRT-Markern als auch mit Röntgen-Markern versehen, wie dies auch in der WO 2009/141165 beschrieben ist, auf die hiermit vollinhaltlich Bezug genommen wird. Als MRT-Marker werden vorzugsweise passiv-negative MRT-Marker verwendet. Passiv-negative MRT-Marker sind paramagnetische, ferromagnetische, ferrimagnetische und antiferromagnetische Metalle, Metalllegierungen und Metallverbindungen. Sie sind vorzugsweise als Partikel in eine Kunststoffmatrix eingebettet. Die passiven MRT-Marker sind vorzugsweise folgende Metalle oder Metallverbindungen: Cobalt (Co), Nickel (Ni), Molybdän (Mo), Zirkonium (Zr), Titan (Ti). Mangan (Mn), Rubidium (Rb), Aluminium (Al), Palladium (Pd), Platin (Pt), Chrom (Cr) oder Chromdioxid (CrO₂), und insbesondere Eisen (Fe) und Eisenoxid (FeO, Fe₂O₃, Fe₃O₄) und Verbindungen oder Legierungen, wie z.B. FePt, aus diesen Metallen. Die Konzentration der passiv-negativen MRT-Marker ist so zu wählen, dass sie bei den gewünschten Sequenzen sichtbar sind, die medizinische Vorrichtung in zumindest einer MRT-Sequenz gut abbilden, aber die Abbildung des umgebenden Körpergewebes dabei nicht überlagern oder stören.

Als Röntgen-Marker werden hingegen folgende Metalle oder andere Elemente Barium (Ba), Wolfram (W), Tantal (Ta), Osmium (Os), Praseodym (Pr), Platin (Pt), Gold (Au) und Blei (Pb) eingesetzt. Diese Elemente können in elementarer Form oder auch in Verbindungen, wie z.B. Bariumsulfat, als Röntgen-Marker verwendet werden.

Die Röntgen-Marker beeinflussen in der Regel die Bildgebung bei einem magnetresonanztomographischen Verfahren kaum. Sie sind jedoch in Röntgenuntersuchungen, z.B. der Computertomographie oder Durchleuchtungen, mittels Röntgenstrahlung gut erkennbar.

Einige Marker können grundsätzlich sowohl als Röntgen-Marker als auch als passivnegativer MRT-Marker verwendet werden, wobei die bildgebende Funktion jeweils von der Konzentration abhängt. Wie es unten noch näher ausgeführt wird, erzeugt Eisen sowohl bei einer Magnetresonanztomographie als auch bei einer Röntgenuntersuchung Bildsignale. Jedoch sind die für die Röntgenuntersuchung notwendigen Eisen-Konzentrationen so hoch, dass hierdurch das Bild bei der Magnetresonanztomographie gestört wird. Marker, die sowohl als Röntgen-Marker als auch als MRT-Marker verwendbar sind, werden in einer solchen Konzentration eingesetzt, dass sie weder die Magnetresonanztomographie noch die Röntgenuntersuchung stören. In der Regel ist die Konzentration dieser Marker so eingestellt, dass sie nur bei der Magnetresonanztomographie ein Bildsignal erzeugen und bei der Röntgen-Untersuchung kaum sichtbar sind. Bei Platin ist die Situation ähnlich, aber hier ist der Unterschied in der Wirkung bei den beiden bildgebenden Verfahren nicht so ausgeprägt.

Der Röntgen-Marker ist aus Partikeln ausgebildet, die in ein Verbundelement bzw. einem stabförmigen Körper eingebettet sind. Das stabförmige Verbundelement ist wiederum Bestandteil der medizinischen Vorrichtung, das mehrere solcher stabförmigen Verbundelemente umfassen kann, die mit den gleichen oder auch unterschiedlichen einschließlich passiv-negativen MRT-Markern versehen sein können. Ein solches stabförmige Verbundelement ist vorzugsweise ausgebildet, wie es als stabförmiger Körper in der WO 2007/000148 A2 beschrieben ist. Auf dieses Dokument wird diesbezüglich Bezug genommen.

Das stabförmige Verbundelement ist schlecht elektrisch leitend ausgebildet. Die Partikel der Marker können grundsätzlich gut elektrisch leitend sein (z.B. Eisen- oder Platin-Partikel). Sie sind jedoch in einer solchen Konzentration vorzusehen, dass sie durch das Matrixmaterial voneinander isoliert sind und zumindest keinen elektrischen Leiter ausbilden, der länger als 15 cm und vorzugsweise nicht länger als 10 cm bzw. 5 cm ist.

Die Verwendung derartiger stabförmiger Verbundelemente, die in der Regel einen Durchmesser von 0,1 mm bis 0,7 mm und vorzugsweise von 0,1 mm bis 0,3 mm aufweisen, erlaubt die einfache Herstellung von Seelen, wobei die Seele auf einfache Weise mit unterschiedlichen Markern ausgebildet werden kann, indem es aus unterschiedlich dotierten stabförmigen Verbundelementen ausgebildet wird. Die stabförmigen Verbundelemente können in ein weiteres übergeordnetes Matrixmaterial, der Hüllmatrix, zur Ausbildung der Seele eingebettet werden. Sie können jedoch auch zur Ausbildung einer Seele geflochten und mit einem Matrixmaterial und/oder einer Hüllmatrix vergossen werden.

Eine solche Hüllmatrix besteht aus einem Hüllpolymer, das nicht durch Filamente verstärkt ist. Grundsätzlich besteht das Bestreben die Seelen möglichst steif auszubilden. Hat die Hüllmatrix keine bildgebende Funktion, dann wird die Seele vorzugsweise aus einem einzigen stabförmigen Körper ohne Hüllmatrix ausgebildet, da das Verbundmaterial bestehend aus nicht-metallischen Filamenten und dem Matrixmaterial eine wesentlich größere Steifigkeit als das Hüllmatrix besitzt.

Eine Seele mit zumindest einem Röntgen-Marker und zumindest einem MR-Marker kann somit sowohl bei einer Röntgenuntersuchung als auch bei einer Magnetresonanztomographieuntersuchung eingesetzt werden und ist jeweils gut sichtbar, ohne dass die Bildgebung durch einen der beiden Marker gestört wird.

### Bezugszeichenliste

- 1.: medizinische Vorrichtung
- 2.: Lead-Sonde
- 3.: proximaler Endbereich
- 4.: distaler Endbereich
- 5.: Öffnung
- 6.: axiale Richtung
- 7.: Lumen
- 8.: Seele
- 9.: nicht-metallische Filamente
- 10.: Matrixwerkstoff
- 11.: Aramidfaser
- 12.: Glasfaser
- 13.: Verbundelement
- 14.: Hüllmatrix

## Patentansprüche

1. Medizinische Vorrichtung zum Einbringen in den menschlichen oder tierischen Körper umfassend,
eine langgestreckte, rohrförmige Lead-Sonde mit einem proximalen und einem distalen Endbereich, wobei am proximalen Ende der Lead-Sonde eine Öffnung ausgebildet ist, die in ein sich in axialer Richtung erstreckendes Lumen mündet, und das distale Ende der Lead-Sonde verschlossen ist,
eine langgestreckte Seele,
wobei die Seele derart ausgebildet ist, dass sie über das Lumen in die Lead-Sonde einbringbar ist,
**dadurch gekennzeichnet,**
**dass** die Seele aus nicht-metallischen Filamenten, die in einen Maxtrixwerkstoff eingebettet sind, ausgebildet ist.

2. Medizinische Vorrichtung nach Anspruch 1,
**dadurch gekennzeichnet,**
**dass** der Durchmesser der Seele in etwa 60% bis 98% und insbesondere 70% bis 95% des Durchmessers des.Lumen der Lead-Sonde beträgt.

3. Medizinische Vorrichtung nach Anspruch 1 oder 2,
**dadurch gekennzeichnet,**
**dass** die Seele eine höhere Biegesteifigkeit als die Lead-Sonde aufweist,
wobei die Seele vorzugsweise Aramidfasern und Glasfasern umfasst.

4. Medizinische Vorrichtung nach einem der Ansprüche 1 bis 3,
**dadurch gekennzeichnet,**
**dass** die Seele einen MRT-Marker aufweist, wobei sich der MRT-Marker insbesondere über einen Grossteil der Länge der Seele erstreckt, wobei vorzugsweise die Seele an ihrem distalen Endbereich mit einem MRT-Marker versehen ist.

5. Medizinische Vorrichtung nach Anspruch 4,
**dadurch gekennzeichnet,**
**dass** der MRT-Marker aus Mikropartikel oder Nanopartikel aus einem der folgenden Metalle oder Metallverbindungen ausgebildet ist: Cobal (Co), Nickel (Ni), Molybdän (Mo), Zirkonium (Zr), Titen /Ti), Mangan (Mn), Rubidium (Rb), Aluminium (Al), Palladium (Pd), Platin (Pt), Chrom (Cr) oder Chromdioxid (Cr02), Eisen (Fe), Eisenoxid (FeO, Fe203, Fe304), FePt, wobei die Mikropartikel vorzugsweise Eisenpartikel sind, die eine Grösse von 1 pm bis 150 pm, insbesondere 20 pm bis 70 pm und vorzugsweise 40 pm bis 60 pm aufweisen.

6. Medizinische Vorrichtung nach Anspruch 4 oder 5,
**dadurch gekennzeichnet,**
**dass** die MRT-Marker entlang der zentrischen Längsachse der Seele konzentriert sind, vorzugsweise, indem sich die Marker in einem zentrisch in der Seele angeordneten langgestreckten Verbundelement befinden.

7. Medizinische Vorrichtung nach einem der Ansprüche 1 bis 6,
**dadurch gekennzeichnet,**
**dass** die Seele Röntgen-Marker aufweist, wobei die Röntgen-Marker vorzugsweise Barium (Ba), Wolfram (W), Tantal (Ta), Osmium (Os), Praseodym (Pr), Platin (Pt), Gold (Au), Blei (Pb) und insbesondere Bariumsulfat umfassen.

8. Medizinische Vorrichtung nach einem der Ansprüche 1 bis 7,
**dadurch gekennzeichnet,**
**dass** die nicht-metallischen Filamente aus Glasfasern, Keramikfasern, Dacron(R), Aramid, Polyaramid, Kevlar(R), Dyneema(R) und/oder pflanzlichen Fasern ausgebildet sind.

9. Medizinische Vorrichtung nach einem der Ansprüche 1 bis 8,
**dadurch gekennzeichnet,**
**dass** die Seele aus einem oder mehreren langgestreckten Verbundelementen ausgebildet ist, wobei ein jedes Verbundelement aus den nicht-metallischen Filamenten, die in den Matrixwerkstoff eingebettet sind, ausgebildet sind, wobei das/die Verbundelement(e) in eine Hüllmatrix eingebettet sind, wobei der Matrixwerkstoff vorzugsweise härter als die Hüllmatrix ist und insbesondere der Matrixwerkstoff aus Epoxidharz und die Hüllmatrix aus Polyurethan (PU), PVC oder Gummi ausgebildet sind.

10. Medizinische Vorrichtung nach einem der Ansprüche 1 bis 9,
**dadurch gekennzeichnet,**
**dass** im proximalen Endbereich der Lead-Sonde eine Elektrode angeordnet ist, so dass die medizinische Vorrichtung eine Herzschrittmachersonde ausbildet.

11. Medizinische Vorrichtung nach einem der Ansprüche 1 bis 10,
**dadurch gekennzeichnet,**
**dass** die medizinische Vorrichtung einen stabförmigen Körper umfasst, der an Stelle der Seele in die Lead-Sonde einführbar ist, wobei der stabförmige Körper eine Strahlenquelle aufweist, wobei vorzugsweise die Lead-Sonde aus einem steifen Material oder aus einem flexiblen Material ausgebildet ist.

12. Verfahren zum Positionieren einer medizinischen Vorrichtung in einem menschlichen oder tierischen Körper, wobei
in den menschlichen oder tierischen Körper eine medizinische Vorrichtung gemäß einem der Ansprüche 1 bis 11 eingeführt wird.

13. Verfahren gemäß Anspruch 12,
**dadurch gekennzeichnet,**
**dass** durch Verschieben der Seele in der Lead-Sonde, wobei die Seele vorzugsweise eine höhere Biegesteifigkeit als die Lead-Sonde aufweist, die Biegesteifigkeit der medizinischen Vorrichtung zumindest im distalen Endbereich verändert wird.

14. Verfahren gemäß Anspruch 12 oder 13,
**dadurch gekennzeichnet,**
**dass** die Seele MRT-Marker und/oder Röntgen-Marker aufweist, wodurch die Position der medizinischen Vorrichtung im menschlichen oder tierischen Körper beim Einführen der medizinischen Vorrichtung mittels einer MRT- oder Röntgenuntersuchung detektiert wird.

15. Verfahren nach einem der Ansprüche 12 bis 14,
**dadurch gekennzeichnet,**
**dass** die medizinische Vorrichtung als Schrittmachersonde oder zur Brachytherapie verwendet wird.

## Claims

1. A medical device for insertion into a human or animal body, comprising
an elongated, tubular lead probe having a proximal end zone and a distal end zone, the proximal end of the lead probe being provided with an aperture opening into a lumen extending in axial direction, and the distal end of the lead probe being closed,
an elongated core,
the core being designed such that it can be inserted into the lead probe via the lumen,
**characterized in that**
the core is made of non-metallic filaments which are embedded in a matrix material.

2. The medical device according to claim 1,
**characterized in that**
the diameter of the core amounts to approximately 60% to 98% and in particular 70% to 95% of the diameter of the lumen of the lead probe.

3. The medical device according to claim 1 or 2,
**characterized in that**
the core has a higher flexural rigidity than the lead probe and preferably comprises aramide fibers and glass fibers.

4. The medical device according to any of claims 1 to 3,
**characterized in that**
the core comprises an MRT marker extending in particular over a major part of the length of the core, the core preferably having its distal end zone provided with an MRT marker.

5. The medical device according to claim 4,
**characterized in that**
the MRT markers is made from microparticles or nanoparticles of one of the following metals or metallic compounds: cobalt (Co), nickel (Ni), molybdenum (Mo), zirconium (Zr), titanium (Ti), manganese (Mn), rubidium (Rb), aluminum (Al), palladium (Pd), platinum (Pt), chromium (Cr) or chromium dioxide (Cr0₂), iron (Fe), iron oxide (FeO, Fe203, Fe304), FePt, the microparticles preferably being iron particles with a size from 1 pm to 150 pm, in particular 20 pm to 70 pm and preferably 40 pm to 60 pm.

6. The medical device according to claim 4 or 5,
**characterized in that**
the MRT markers are concentrated along the centric longitudinal axis of the core, preferably by the markers being situated in an elongated composite element centrically arranged in the core.

7. The medical device according to any of claims 1 to 6,
**characterized in that**
the core comprises X-ray markers, the X-ray markers preferably comprising barium (Ba), tungsten (W), tantalum (Ta), osmium (Os), praseodymium (Pr), platinum (Pt), gold (Au), lead (Pb) and in particular barium sulfate.

8. The medical device according to any of claims 1 to 7,
**characterized in that**
the non-metallic filaments are made of glass fibers, ceramic fibers, Dacron®, aramide, polyaramide, Kevlar®, Dyneema® and/or vegetable fibers.

9. The medical device according to any of claims 1 to 8,
**characterized in that**
the core is made of one or more elongated composite elements, with each of the composite elements being made of the non-metallic filaments which are embedded in the matrix material, the composite elements(s) being embedded in a sheathing matrix, the matrix material preferably having a hardness which is higher than that of the sheathing matrix and, in particular, the matrix material being made of epoxy resin and the sheathing matrix being made from polyurethane (PU), PVC or rubber.

10. The medical device according to any of claims 1 to 9,
**characterized in that**
an electrode is arranged in the proximal end zone of the lead probe, so that the medical device forms a pacemaker probe.

11. The medical device according to any of claims 1 to 10,
**characterized in that**
the medical device comprises a rod-shaped body which can be inserted into the lead probe in place of the core and comprises a radiation source, the lead probe being preferably made of a rigid material or a flexible material.

12. A method of positioning a medical device in a human or animal body, wherein
a medical device according to any of claims 1 to 11 is inserted into the human or animal body.

13. The method according to claim 12,
**characterized in that**
the flexural rigidity of the medical device is altered at least in the distal end zone by shifting the core in the lead probe, the core preferably having a higher flexural rigidity than the lead probe.

14. The method according to claim 12 or 13,
**characterized in that**
the core comprises MRT markers and/or X-ray markers whereby the position of the medical device in the human or animal body during the insertion of the medical device is detected by means of an MRT or X-ray examination.

15. The method according to any of claims 12 to 14,
**characterized in that**
the medical device is used as a pacemaker probe or for brachytherapy.

## Revendications

1. Dispositif médical pour introduction dans le corps humain ou animal, comprenant,
une sonde tubulaire allongée avec une zone d'extrémité proximale et une zone d'extrémité distale, à l'extrémité proximale de la sonde étant réalisée une ouverture qui débouche dans une lumière s'étendant dans le sens axial, et l'extrémité distale de la sonde étant fermée,
une âme allongée,
dans lequel l'âme est réalisée de sorte qu'elle puisse être introduite à travers la lumière dans la sonde,
**caractérisé par le fait**
**que** l'âme est réalisée à partir de filaments non métalliques noyés dans un matériau de matrice.

2. Dispositif médical selon la revendication 1,
**caractérisé par le fait**
**que** le diamètre de l'âme est d'environ 60% à 98% et en particulier de 70% à 95% du diamètre de la lumière de la sonde.

3. Dispositif médical selon la revendication 1 ou 2,
**caractérisé par le fait**
**que** l'âme présente une plus grande résistance à la flexion que la sonde,
l'âme comprend de préférence des fibres d'aramide et des fibres de verre.

4. Dispositif médical selon l'une des revendications 1 à 3,
**caractérisé par le fait**
**que** l'âme présente un marqueur pour l'IRM, le marqueur pour l'IRM s'étendant en particulier sur une grande partie de la longueur de l'âme, l'âme étant de préférence pourvue à son extrémité distale d'un marqueur pour l'IRM.

5. Dispositif médical selon la revendication 4,
**caractérisé par le fait**
**que** le marqueur pour l'IRM est composé de microparticules ou de nanoparticules de l'un des métaux ou composés métalliques suivants: cobalt (Co), nickel (Ni), molybdène (Mo), zirconium (Zr), titane (Ti), manganèse (Mn), rubidium (Rb), aluminium (Al), palladium (Pd), platine (Pt), chrome (Cr) ou dioxyde de chrome (Cr02), fer (Fe), oxyde de fer (FeO, Fe203, Fe304), FePt, les microparticules étant de préférence des particules de fer présentant une grosseur de 1 pm à 150 pm, en particulier de 20 pm à 70 pm et de préférence de 40 pm à 60 pm.

6. Dispositif médical selon la revendication 4 ou 5,
**caractérisé par le fait**
**que** les marqueurs pour l'IRM sont concentrés le long de l'axe longitudinal central de l'âme, de préférence en ce que les marqueurs se trouvent dans un élément composite allongé disposé centralement dans l'âme.

7. Dispositif médical selon l'une des revendications 1 à 6,
**caractérisé par le fait**
**que** l'âme présente des marqueurs à rayons X, les marqueurs à rayons X comprenant de préférence du baryum (Ba), du tungstène (W), du tantale (Ta), de l'osmium (Os), du praséodyme (Pr), du platine (Pt), de l'or (Au), du plomb (Pb) et en particulier du sulfate de baryum.

8. Dispositif médical selon l'une des revendications 1 à 7,
**caractérisé par le fait**
**que** les filaments non métalliques sont réalisés en fibres de verre, en fibres céramiques, en Dacron(R), en aramide, en polyaramide, en kevlar(R), en dyneema(R) et/ou en fibres végétales.

9. Dispositif médical selon l'une des revendications 1 à 8,
**caractérisé par le fait**
**que** l'âme est réalisée à partir d'un ou plusieurs éléments composites allongés, chaque élément composite étant réalisé à partir des filaments non métalliques qui sont noyés dans le matériau de matrice, le ou les éléments composites étant noyés dans une matrice de revêtement, le matériau de matrice étant de préférence plus dur que la matrice de revêtement, et en particulier le matériau de matrice est réalisé en résine époxyde et la matrice de revêtement étant réalisée en polyuréthane (PU), en PVC ou en caoutchouc.

10. Dispositif médical selon l'une des revendications 1 à 9,
**caractérisé par le fait**
**que** dans la zone d'extrémité proximale de la sonde est disposée une électrode, de sorte que le dispositif médical forme une sonde de stimulation cardiaque.

11. Dispositif médical selon l'une des revendications 1 à 10,
**caractérisé par le fait**
**que** le dispositif médical comporte un corps en forme de tige qui peut être introduit au lieu de l'âme dans la sonde, le corps en forme de tige présentant une source de rayonnement, la sonde étant réalisée de préférence en un matériau rigide ou un matériau souple.

12. Procédé pour positionner un dispositif médical dans un corps humain ou animal, dans lequel
dans le corps humain ou animal est introduit un dispositif médical selon l'une des revendications 1 à 11.

13. Procédé selon la revendication 12,
**caractérisé par le fait**
**que** par déplacement de l'âme dans la sonde, l'âme présentant de préférence une plus grande résistance à la flexion que la sonde, est changée la résistance à la flexion du dispositif médical au moins dans la zone d'extrémité distale.

14. Procédé selon la revendication 12 ou 13,
**caractérisé par le fait**
**que** l'âme présente des marqueurs pour l'IRM et/ou des marqueurs pour rayons X, d'où la position du dispositif médical dans le corps humain ou animal est détectée, lors de l'introduction du dispositif médical, au moyen d'un IRM ou d'un examen par rayons X.

15. Procédé selon l'une des revendications 12 à 14,
**caractérisé par le fait**
**que** le dispositif médical est utilisé comme sonde de stimulation ou pour la curiethérapie.
